(19) Europäisches Patentamt
European Patent Office
Office européen des brevets

(11) Veröffentlichungsnummer: **0 138 085**
**A1**

# (12) EUROPÄISCHE PATENTANMELDUNG

(21) Anmeldenummer: **84111094.3**

(22) Anmeldetag: **18.09.84**

(51) Int. Cl.⁴: **C 07 D 303/08**, A 01 N 43/20
// C07C25/13, C07C25/24

(30) Priorität: **30.09.83 DE 3335477**

(43) Veröffentlichungstag der Anmeldung: **24.04.85 Patentblatt 85/17**

(84) Benannte Vertragsstaaten: **AT BE CH DE FR GB IT LI NL**

(71) Anmelder: **BAYER AG, Konzernverwaltung RP Patentabteilung, D-5090 Leverkusen 1 Bayerwerk (DE)**

(72) Erfinder: **Lantzsch, Reinhard, Dr., Heymannstrasse 32, D-5090 Leverkusen 1 (DE)**
Erfinder: **Hagemann, Hermann, Dr., Kandinskystrasse 52, D-5090 Leverkusen 1 (DE)**
Erfinder: **Eue, Ludwig, Dr., Paul-Klee-Strasse 36, D-5090 Leverkusen 1 (DE)**
Erfinder: **Schmidt, Robert R. Dr., Im Waldwinkel 110, D-5060 Bergisch-Gladbach 2 (DE)**

(54) **2-Aryl-2-Halogenalkyloxirane.**

(57) Die Erfindung betrifft neue 2-Aryl-2-halogenalkyloxirane der Formel (I)

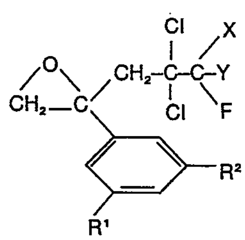

(I)

in welcher
X und Y unabhängig voneinander für Fluor oder Chlor stehen und
$R^1$ und $R^2$ unabhängig voneinander für Halogen, Alkyl oder Halogenalkyl stehen,
ein Verfahren zu ihrer Herstellung und ihre Verwendung als Herbizide.

EP 0 138 085 A1

BAYER AKTIENGESELLSCHAFT
Konzernverwaltung RP
Patentabteilung

5090 Leverkusen, Bayerwerk

Bi/by-c
Ia

## 2-Aryl-2-halogenalkyloxirane

Die Erfindung betriftt neue 2-Aryl-2-halogenalkyloxirane, ein Verfahren zu ihrer Herstellung und ihre Verwendung als Herbizide, insbesondere als selektive Herbizide.

Es ist bereits bekannt, daß bestimmte 2-Aryl-2-halogenalkyloxirane, wie beispielsweise das 2-(3,5-Dichlorphenyl)-2-(2,2,2-trichlorethyl)-oxiran herbizide Eigenschaften besitzen (vgl. DE-OS 25 19 073 bzw. US-PS 4 211 549). In bestimmten Nutzpflanzenkulturen ist deren Einsatz jedoch nicht möglich, da es infolge der hohen herbiziden Potenz dieser Stoffe auch bei bestimmten Nutzpflanzen zu Schäden kommen kann; die Verträglichkeit gegenüber den vorbekannten 2-Aryl-2-halogenalkyloxiranen ist demnach bei verschiedenen Nutzpflanzen nicht ausreichend.

Es wurden neue 2-Aryl-2-halogenalkyloxirane der allgemeinen Formel (I)

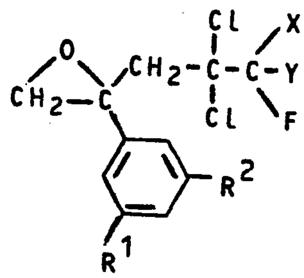

(I)

in welcher

X und Y unabhängig voneiander für Fluor oder Chlor stehen und

$R^1$ und $R^2$ unabhängig voneinander für Halogen, Alkyl oder Halogenalkyl stehen,

gefunden.

Weiterhin wurde gefunden, daß man die neuen 2-Aryl-2-halogenalkyloxirane der allgemeinen Formel (I),

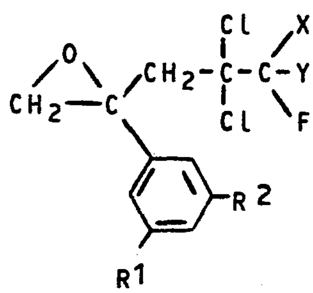

(I)

in welcher

X und Y unabhängig voneinander für Fluor oder Chlor stehen und

$R^1$ und $R^2$ unabhängig voneinander für Halogen, Alkyl oder Halogenalkyl stehen,

erhält, wenn man

substituierte Styrol-Derivate der Formel (II)

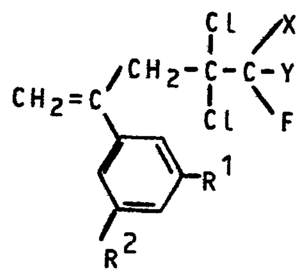

(II)

in welcher

$R^1$, $R^2$, X und Y die oben angegebene Bedeutung haben,

mit Oxidationsmitteln der Formel (III)

R-O-O-H (III)

in welcher

R für Wasserstoff oder für gegebenenfalls substituiertes Alkylcarbonyl oder Arylcarbonyl steht,

gegebenenfalls in Gegenwart eines Verdünnungsmittels und gegebenenfalls in Gegenwart eines Säurebindemittels umsetzt.

Schließlich wurde gefunden, daß die neuen 2-Aryl-2-halogenalkyloxirane der Formel (I) gute herbizide, insbesondere selektiv-herbizide Eigenschaften besitzen.

Dabei zeigen die erfindungsgemäßen Stoffe überraschenderweise bei gleich guter allgemein-herbizider Wirkung eine deutlich bessere Verträglichkeit gegenüber wichtigen Kulturpflanzen, wie insbesondere Mais, Soja und Zuckerrüben, als das aus dem Stand der Technik bekannte 2-(3,5-Dichlorphenyl)-2-(2,2,2-trichlorethyl)-oxiran, welches eine chemisch und wirkungsmäßig naheliegende Verbindung ist.

Die erfindungsgemäßen 2-Aryl-2-halogenalkyloxirane sind durch die Formel (I) allgemein definiert. Bevorzugt sind Verbindungen, bei denen

X und Y unabhängig voneinander für Fluor oder Chlor stehen und

$R^1$ und $R^2$ unabhängig voneinander für Fluor, Chlor, Brom, geradkettiges oder verzweigtes Alkyl mit bis zu 4 Kohlenstoffatomen oder für geradkettiges oder verzweigtes Halogenalkyl mit bis zu 4 Kohlenstoffatomen und bis zu 4 gleichen oder verschiedenen Halogenatomen stehen.

Besonders bevorzugt sind Verbindungen der Formel (I), in welcher

X und Y unabhängig voneinander für Fluor oder Chlor stehen und

$R^1$ und $R^2$ unabhängig voneinander für Fluor, Chlor, Brom, Methyl, Ethyl, n- und i-Propyl oder Trifluormethyl stehen.

Im einzelnen seien außer den bei den Herstellungsbeispielen genannten Verbindungen die folgenden Verbindungen der allgemeinen Formel (I) genannt:

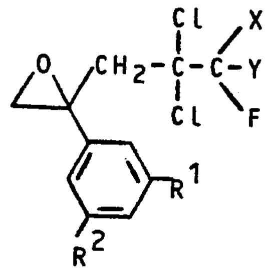

(I)

| $R^1$ | $R^2$ | X | Y |
|---|---|---|---|
| F | F | F | F |
| F | F | Cl | F |
| F | F | Cl | Cl |
| Br | Br | F | F |
| Br | Br | Cl | F |
| Br | Br | Cl | Cl |
| $CH_3$ | $CH_3$ | F | Cl |
| $CH_3$ | $CH_3$ | Cl | Cl |
| $CF_3$ | $CF_3$ | F | F |
| $CF_3$ | $CF_3$ | Cl | F |
| $CF_3$ | $CF_3$ | Cl | Cl |
| $CF_3$ | $i-C_3H_7$ | Cl | F |
| $CF_3$ | $i-C_3H_7$ | F | F |
| $CF_3$ | $i-C_3H_7$ | Cl | Cl |

Verwendet man als Ausgangsstoffe beispielsweise 3,5-Dichlor-α-(2,2-dichlor-3,3,3-trifluor-prop-1-yl)-styrol und m-Chlorperbenzoesäure, so läßt sich die Durchführung des erfindungsgemäßen Verfahrens durch das folgende Formelschema darstellen:

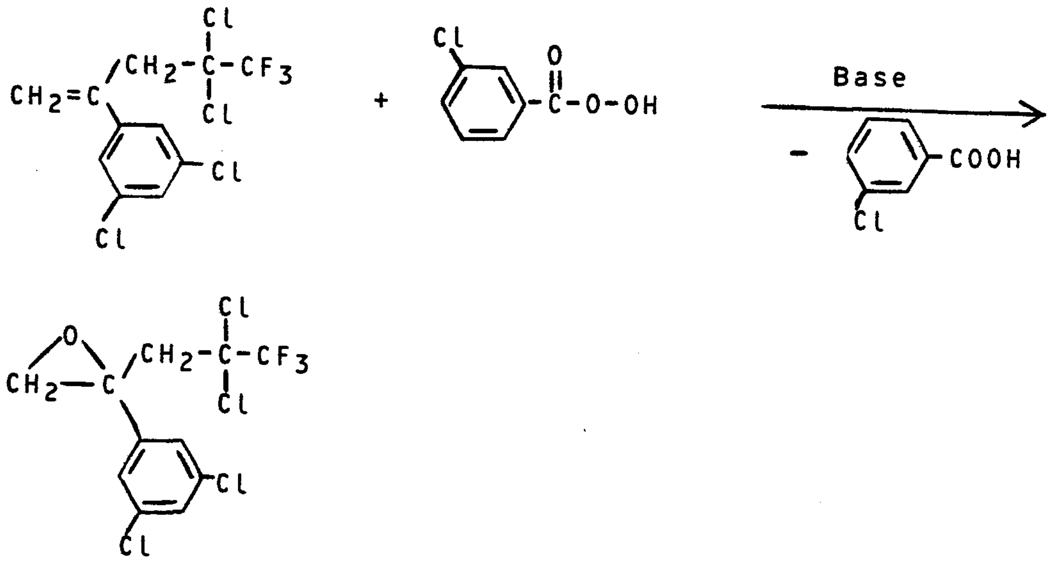


Die zur Durchführung des erfindungsgemäßen Verfahrens als Ausgangsstoffe benötigten substituierten Styrol-Derivate sind durch die Formel (II) allgemein definiert. In dieser Formel (II) stehen X, Y, $R^1$ und $R^2$ vorzugsweise für diejenigen Reste, die schon bei der Beschreibung der erfindungsgemäßen Stoffe der Formel (I) als bevorzugt für diese Substituenten angegeben wurden.

Die substituierten Styrol-Derivate der Formel (II) sind noch nicht bekannt. Man erhält sie jedoch in prinzipiell bekannter Weise (vgl. z.B. DE-OS 2 831 193), wenn man

allgemein bekannte α-Methylstyrol-Derivate der Formel (IV)

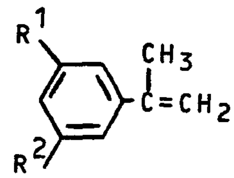

(IV)

in welcher

$R^1$ und $R^2$ die oben angegebene Bedeutung haben,

mit Perhalogenethanen der Formel (V)

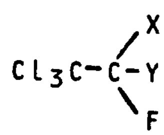

(V)

in welcher

X und Y die oben angegebene Bedeutung haben,

zunächst in einer 1. Stufe, gegebenenfalls in Gegenwart eines Verdünnungsmittels, wie beispielsweise Acetonitril, gegebenenfalls in Gegenwart eines Katalysators wie beispielsweise Benzoylperoxid oder Kupfer-I-chlorid/Cyclohexylamin oder des Redox-Systems Eisen-III-chlorid-Hexahydrat, Benzoin und Dimethylamin-Hydrochlorid bei Temperaturen zwischen 70°C und 180°C und den Druckbereichen zwischen 1 bar und 15 bar umsetzt zu den Verbindungen der Formel (VI)

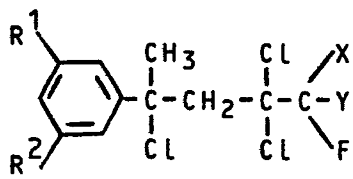

(VI)

in welcher

$R^1$,$R^2$,X und Y die oben angegebene Bedeutung haben,

und diese in einer 2. Stufe, ebenfalls in prinzipiell bekannter Weise mit katalytischen Mengen einer Lewis-Säure, wie beispielsweise Antimon-pentachlorid, gegebenenfalls in Gegenwart eines Verdünnungsmittels wie beispielsweise Tetrachlorkohlenstoff bei Temperaturen zwischen 20°C und 150°C dehydrochloriert.

Die weiterhin zur Durchführung des erfindungsgemäßen Verfahrens als Ausgangsstoffe benötigten Oxidationsmittel sind durch die Formel (III) allgemein definiert. In dieser Formel (III) steht R vorzugsweise für Wasserstoff, für Acetyl, für Propionyl, für Trifluoracetyl oder für gegebenenfalls substituiertes Benzoyl, wie beispielsweise 3-Chlorbenzoyl oder 4-Nitrobenzoyl. Die als Oxidationsmittel zu verwendenden Peroxyverbindungen sind bekannt.

Für das erfindungsgemäße Verfahren kommen als Verdünnungsmittel inerte organische Lösungsmittel infrage. Vorzugsweise verwendet man Kohlenwasserstoffe, wie Benzin, Benzol, Toluol, Xylol, Hexan oder Petrolether,

chlorierte Kohlenwasserstoffe, wie Dichlormethan, 1,2-Dichlorethan, Chloroform, Tetrachlorkohlenstoff oder Chlorbenzol, Ether, wie Diethylether, Dioxan oder Tetrahydrofuran, Carbonsäuren, wie Essigsäure oder Propionsäure, oder dipolar aprotische Lösungsmittel wie Acetonitril, Aceton, Essigsäureethylester oder Dimethylformamid.

Das erfindungsgemäße Verfahren wird gegebenenfalls in Gegenwart eines Säurebindemittels durchgeführt. Als solche kommen alle üblicherweise verwendbaren organischen und anorganischen Säurebindemittel infrage. Vorzugsweise verwendet man Erdalkali- oder Alkalimetallhydroxide, -acetate oder -carbonate, wie beispielsweise Calciumhydroxid, Natriumhydroxid, Natriumacetat oder Natriumcarbonat.

Die Reaktionstemperaturen können bei dem erfindungsgemäßen Verfahren in gewissen Bereichen variiert werden. Im allgemeinen arbeitet man zwischen -20°C und +70°C, vorzugsweise zwischen 0°C und 50°C.

Zur Durchführung des erfindungsgemäßen Verfahrens setzt man pro Mol des substituierten Styrol-Derivates der Formel (II) im allgemeinen 0,8 bis 1,5 Mol, vorzugsweise äquimolare Mengen an Oxidationsmittel der Formel (III) ein. Die Aufarbeitung und Isolierung der Reaktionsprodukte der Formel (I) erfolgt in allgemein üblicher Art und Weise.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

<u>Monokotyle Kulturen der Gattungen:</u> Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe zeigen neben einer sehr guten allgemeinen herbiziden Wirkung eine hohe Verträglichkeit gegenüber wichtigen Kulturpflanzen. So ist es möglich, schwer bekämpfbare Schadgräser in Kulturen von Baumwolle, Erdnüssen, Kartoffeln, Raps, Sojabohnen, Zuckerrüben und Mais selektiv zu bekämpfen.

Die Wirkstoffe können in die üblichen Formulierungen überführt werden, wie Lösungen, Emulsionen, Spritzpulver, Suspensionen, Pulver, Stäubemittel, Pasten, lösliche

Pulver, Granulate, Suspensions-Emulsions-Konzentrate, wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z. B. Ammoniumsalze und natürliche Gesteinsmehle, wie Kaoline, Tonerden, Talkum, Kreide, Quarz, Attapulgit,

0138085

Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,1 und 15 kg Wirkstoff pro Hektar Bodenfläche, vorzugsweise zwischen 0,5 und 10 kg pro ha.

Die Herstellung und die Verwendung der erfindungsgemäßen Wirkstoffe geht aus den nachfolgenden Beispielen hervor.

## Herstellungsbeispiele

### Beispiel 1

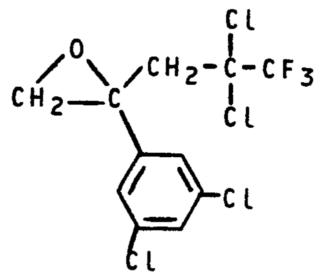

12,5 g (0,037 Mol) 3,5-Dichlor-$\alpha$-(2,2-dichlor-3,3,3-trifluorprop-1-yl)-styrol werden in 120 ml Dichlormethan gelöst und anschließend mit 16 g (0,15 Mol) feingepulvertem Natriumcarbonat sowie 6,8 g (0,04 Mol) m-Chlorperbenzoesäure versetzt. Die Mischung wird 30 Stunden bei 40°C gerührt. Nach dem Abkühlen filtriert man vom ungelösten Rückstand und engt das Filtrat in Vakuum ein. Der Rückstand wird im Hochvakuum destilliert.

Man erhält 8,5 g (73,9 % der Theorie) an 2-(3,5-Dichlorphenyl)-2-(2,2-dichlor-3,3,3-trifluor-prop-1-yl)-oxiran vom Siedepunkt 115°C bei 0,1 mbar und vom Brechungsindex $n_D^{20}$ = 1,522.

### Herstellung der Ausgangsverbindung

a) 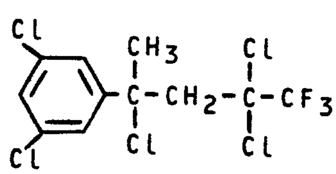

In einem 3 l Emaille-Autoklav gibt man bei 150°C 800 g (4,25 Mol) 3,5-Dichlor- α -methylstyrol und 64 g (0,65 Mol) Cyclohexylamin zu einer Mischung von 1600 g (8,5 Mol) 1,1,1-Trifluor-2,2,2-trichlorethan und 16 g Kupfer-I-chlorid. Man erwärmt 3 Stunden lang auf 140°C bis 150°C, wobei sich ein Druck von 10 bar bis 13 bar einstellt. Die abgekühlte Reaktionsmischung wird filtriert und das Filtrat wird fraktioniert destilliert.

Man erhält 1138 g (71 % der Theorie) an 2-(3,5-Dichlorphenyl)-2,4,4-trichlor-5,5,5-trifluorpentan vom Siedepunkt 96°C bis 102°C bei 0,1 mbar.

b) 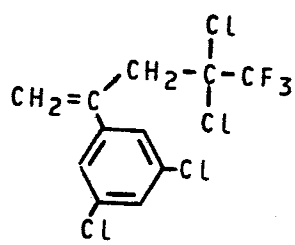

85 g (0,226 Mol) 2-(3,5-Dichlorphenyl)-2,4,4-trichlor-5,5,5-trifluorpentan werden in 70 ml Tetrachlorkohlenstoff tropfenweise mit 2 ml Antimonpentachlorid versetzt, wobei eine Chlorwasserstoffentwicklung einsetzt. Nach beendeter Zugabe erwärmt man für 30 Minuten auf 80°C, wäscht die erhaltene Reaktionsmischung mit 150 ml 10-%iger Salzsäure und mehrmals mit Wasser, trocknet über Natriumsulfat, entfernt das Lösungsmittel im

Vakuum und destilliert den Rückstand im Hochvakuum. Man erhält 69,5 g (91 % der Theorie) an 3,5-Dichlor-α-(2,2-dichlor-3,3-trifluorprop-1-yl)-styrol vom Siedepunkt 105°C bei 0,1 mbar.

In entsprechender Weise und gemäß den allgemeinen Herstellungsangaben erhält man die folgenden Verbindungen der Formel (I):

Beispiel 2 :

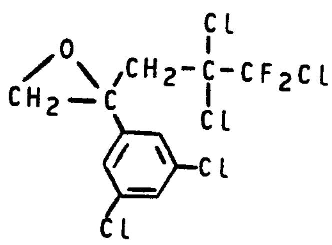

Siedepunkt : 160°C/0,1 mbar

$n_D^{20}$ 1.546

Beispiel 3 :

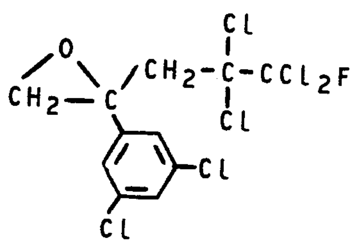

$n_D^{20}$ 1.562

Beispiel 4 :

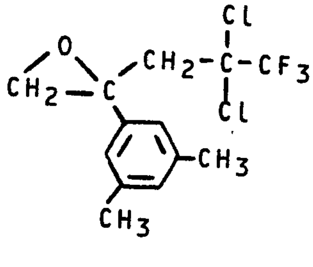

$n_D^{20}$ 1.498

Anwendungsbeispiele

In den folgenden Anwendungsbeispielen wurde die nachstehend aufgeführte Verbindung als Vergleichssubstanz eingesetzt:

(A) 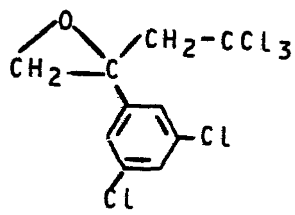

2-(3,5-Dichlorphenyl)-2-(2,2,2-trichlorethyl)-oxiran (bekannt aus DE-OS 25 19 073 bzw. US-PS 4 211 549).

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigt z.B. die Verbindung gemäß Herstellungsbeispiel (1) bei gleich hoher herbizider Wirksamkeit eine bessere Verträglichkeit bei Kulturpflanzen gegenüber der aus dem Stand der Technik bekannten Verbindung (A).

Patentansprüche

1. 2-Aryl-2-halogenalkyl-oxirane der allgemeinen Formel (I)

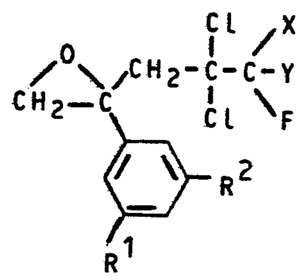

(I)

in welcher

X und Y unabhängig voneinander für Fluor oder Chlor stehen und

$R^1$ und $R^2$ unabhängig voneinander für Halogen, Alkyl oder Halogenalkyl stehen.

2. 2-Aryl-2-halogenalkyl-oxirane der Formel (I) gemäß Anspruch 1, dadurch gekennzeichnet, daß

X und Y unabhängig voneinander für Fluor oder Chlor stehen und

$R^1$ und $R^2$ unabhängig voneinander für Fluor, Chlor, Brom, geradkettiges oder verzweigtes Alkyl mit bis zu 4 Kohlenstoffatomen oder für geradkettiges oder verzweigtes Halogenalkyl mit bis zu 4 Kohlenstoffatomen und bis zu 4 gleichen oder verschiedenen Halogenatomen stehen.

3. 2-Aryl-2-halogenalkyl-oxirane der Formel (I) gemäß Anspruch 1, dadurch gekennzeichnet, daß

X und Y unabhängig voneinander für Fluor oder Chlor stehen und

$R^1$ und $R^2$ unabhängig voneinander für Fluor, Chlor, Brom, Methyl, Ethyl, n- und i-Propyl oder Trifluormethyl stehen.

4. 2-(3,5-Dichlorphenyl)-2-(2,2-dichlor-3,3,3-trifluor-prop-1-yl)-oxiran gemäß Anspruch 1.

5. Verfahren zur Herstellung von 2-Aryl-2-halogenalkyl-oxiranen der Formel (I) gemäß Anspruch 1, dadurch gekennzeichnet, daß man substituierte Styrol-Derivate der Formel (II)

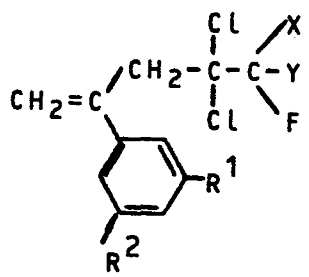

(II)

in welcher

$R^1$,$R^2$,X und Y die oben angegebene Bedeutung haben,

mit Oxidationsmittel der Formel (III)

$$R\text{-}O\text{-}O\text{-}H \qquad (III)$$

in welcher

R für Wasserstoff oder für gegebenenfalls substituiertes Alkylcarbonyl oder Arylcarbonyl steht,

gegebenenfalls in Gegenwart eines Verdünnungsmittels und gegebenenfalls in Gegenwart eines Säurebindemittels umsetzt.

6. Herbizide Mittel, gekennzeichnet durch einen Gehalt an mindestens einem 2-Aryl-2-halogenalkyl-oxiran der Formel (I) gemäß Anspruch 1.

7. Verwendung von 2-Aryl-2-halogenalkyl-oxiranen der Formel (I) gemäß Anspruch 1 zur Bekämpfung von unerwünschtem Pflanzenwachstum.

8. Verfahren zur Herstellung von herbiziden Mitteln, dadurch gekennzeichnet, daß man 2-Aryl-2-halogenalkyl-oxirane der Formel (I) gemäß Anspruch 1 mit Streckmitteln und/oder oberflächenaktiven Mitteln vermischt.

European Patent Office

# EUROPEAN SEARCH REPORT

0138085

Application number

EP 84 11 1094

| Category | DOCUMENTS CONSIDERED TO BE RELEVANT<br>Citation of document with indication, where appropriate, of relevant passages | Relevant to claim | CLASSIFICATION OF THE APPLICATION (Int. Cl.4) |
|---|---|---|---|
| D,A | DE-A-2 519 073 (DOW CHEMICAL)<br>*The whole document*<br>----- | 1-8 | C 07 D 303/08<br>A 01 N 43/20 //<br>C 07 C 25/13<br>C 07 C 25/24 |
| | | | TECHNICAL FIELDS SEARCHED (Int. Cl.4) |
| | | | C 07 D 303/00<br>A 01 N 43/00 |

The present search report has been drawn up for all claims

| Place of search | Date of completion of the search | Examiner |
|---|---|---|
| THE HAGUE | 21-12-1984 | ALLARD M.S. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82